# EUROPÄISCHE PATENTANMELDUNG

(11) **EP 1 479 664 A2**
(43) Veröffentlichungstag der Anmeldung: **24.11.2004**
(21) Anmeldenummer: 04006751.4
(22) Anmeldetag: 20.03.2004
(51) Int. Cl.: C07C 6/04, C07C 11/06

(54) **Verfahren zur Herstellung von Olefinen durch Metathese an einem Carbid oder Oxicarbid eines Nebengruppenmetalls**

(30) Priorität: 30.04.2003 DE 10319437
(71) Anmelder: BASF AKTIENGESELLSCHAFT, 67056 Ludwigshafen (DE)
(72) Erfinder: Schubert, Markus, Dr., 67063 Ludwigshafen (DE); Gerlach, Till, Dr., 67071 Ludwigshafen (DE); Hesse, Michael, Dr., 67549 Worms (DE); Stephan, Jürgen, Dr., 68163 Mannheim (DE); Böhm, Volker, Dr., 67227 Frankenthal (DE); Brodhagen, Andreas, Dr., 67125 Dannstadt-Schauernheim (DE); Poplow, Frank, Dr., 67065 Ludwigshafen (DE)

(57) **Zusammenfassung**

Verfahren zur Herstellung von einer Verbindung mit einer nicht-aromatischen C-C-Doppel oder Dreifachbindung (Verbindung A) aus einer anderen Verbindung oder einer Mischung anderer Verbindungen mit einer nicht-aromatischen C-C-Doppel oder Dreifachfindung (Verbindung B), wobei man die Verbindung (B) bei einer Temperatur von 50 bis 500°C mit einem heterogenen Katalysator, umfassend Carbide oder Oxycarbide eines Nebengruppenelements, in Kontakt bringt.

## Beschreibung

Die Erfindung betrifft ein Verfahren zur Herstellung von einer Verbindung mit einer nicht-aromatischen C-C-Doppel oder Dreifachbindung (Verbindung A) aus einer anderen Verbindung oder einer Mischung anderer Verbindungen mit einer nicht-aromatischen C-C-Doppel oder Dreifachfindung (Verbindung B), wobei man die Verbindung (B) bei einer Temperatur von 50 bis 500°C mit einem heterogenen Katalysator, umfassend Carbide oder Oxycarbide eines Nebengruppenelements, in Kontakt bringt.

Im Text steht die Bezeichnung "heterogener Katalysator, umfassend Carbide oder Oxycarbide eines Nebengruppenelements" auch für einen Katalysator, bei dem verschiedene Nebengruppenelemente nebeneinander vorliegen. Oxide und Carbiden können in einem heterogenen Katalysator ebenfalls nebeneinander vorliegen.

Die Metathese von ungesättigten Verbindungen ist eine allgemein etablierte Methode, um C-C-Verbindungen aufzubrechen und neu zu knüpfen (z. B. Mol, J. C., Chapt. 4.12.2 _{"}Alkene Metathesis" in _{"}Handbook of Heterogeneous Catalysis", Eds. Ertl, G., Knözinger, H., Weitkamp, J., VCH, Weinheim 1997; Weissermehl, K., Arpe, H.-J., Chapt. 3.4 _{"}Olefin-Metathese" in _{"}Industrielle Organische Chemie", 4. Aufl., VCH, Weinheim 1994). Es wurden zahlreiche homogene Katalysatoren entwickelt, hier stellt sich jedoch vor allem die Problematik der Abtrennung des Katalysators aus dem Reaktionsgemisch. Auch für die heterogen katalysierte Metathese sind verschiedene Typen von Katalysatoren beschrieben. Für den Temperaturbereich bis zu ca. 120°C ist die Verwendung geträgerter Re₂O₇- oder Re(CO)₁₀-Katalysatoren üblich (Mol, J. C., Chapt. 4.12.2 "Alkene Metathesis" in "Handbook of Heterogeneous Catalysis", Eds. Ertl, G., Knözinger, H., Weitkamp, J., VCH, Weinheim 1997). Rhenium ist jedoch ein seltenes und relativ teures Element, so dass der Einsatz eines solchen Katalysators oftmals unwirtschaftlich ist. Bei etwas höheren Temperaturen von bis zu 400°C können der Literatur zufolge Katalysatoren auf Basis MoO₃, CoO-MoO₃, MoS₂, Mo(CO)₆ oder diverse geträgerte Mo-Komplexe angewandt werden, bei noch höheren Temperaturen von bis zu 540°C auch Systeme auf Basis WO₃, WS₂, W(CO)₆ oder geträgerte W-Komplexe (Mol, J. C., Chapt. 4.12.2 "Alkene Metathesis" in "Handbook of Heterogeneous Catalysis", Eds. Ertl, G., Knözinger, H., Weitkamp, J., VCH, Weinheim 1997; Weissermehl, K., Arpe, H.-J., Chapt. 3.4 _{"}Olefin-Metathese" in _{"}Industrielle Organische Chemie", 4. Aufl., VCH, Weinheim 1994; Heckelsberg, L. F., Banks, R. L., Bailey, G. C., Ind. Eng. Chem. Prod. Res. Develop. 8 (1969), 259 - 261).

Diese sind zwar sehr preiswert, weisen jedoch generell eine deutlich geringere Aktivität auf und zeigen zudem niedrigere Selektivitäten. Die reduzierten Selektivitäten sind eine Folge der Doppelbindungsisomerisierung, die an den stark sauren Molybdän- und Wolframverbindungen bei höheren Reaktionstemperaturen parallel zur Metathese abläuft, was zur Bildung unerwünschter Produkte führt.

Um die Nebenreaktion der Doppelbindungsisomerisierung zu unterdrücken, wird in US 3,586,731 der Zusatz von Alkali- oder Erdalkalisalzen zu Silca-geträgerten Oxiden, Sulfiden oder Hexacarbonylen von Wolfram, Molybdän oder Rhenium beschrieben. Dies kann allerdings zu einem beträchtlichen Absinken der Katalysatoraktivität führen. In US 4,024,201 wird vorgeschlagen, dem Feed für einen geträgerten WO₃-Katalysator halogenhaltige Verbindungen oder Amine zuzusetzen. Solche polaren Verbindungen sind jedoch auch gleichzeitig als Katalysatorgifte in der Metathese bekannt, daher ist auch hier eine stark erniedrigte Aktivität zu erwarten.

Carbide von Nebengruppenmetallen sind bereits als aktive Katalysatoren für die Skelettisomerisierung von Kohlenwasserstoffen vorbeschrieben (Pham-Huu, C. et al., Ind. Eng. Chem. Res. 34 (1995), 1107; Ribeiro, F. H. et al., J. Catal. 130 (1991), 86). Die Herstellung erfolgt beispielsweise durch Carbidisierung eines Katalysatorprecursors, etwa eines SiO₂-geträgertes Mo- oder W-oxids, in einem kohlenwasserstoffhaltigen Strom unter reduzierenden Bedingungen bei hohen Temperaturen. Verschiedene Herstellmethoden für Carbid-Katalysatoren sind z. B. in Oyama, S. T., Catal. Today 15 (1992), 179 angegeben.

Es bestand deshalb die Aufgabe, ein Verfahren zur Herstellung von ungesättigten Verbindungen, insbesondere Olefine, durch Metathese bereitzustellen, bei dem die gewünschten ungesättigten Verbindungen mit hohem Umsatz und hoher Selektivität gebildet werden und die Nachteile der vorstehenden Verfahren zumindest teilweise vermieden werden. Insbesondere sollen die Katalysatoren schon bei relativ niedrigen Temperaturen eine hohe Metathese-Aktivität aufweisen.

Demgemäß wurde das eingangs definierte Verfahren gefunden.

Für das erfindungsgemäße Verfahren geeignete heterogene Katalysatoren, umfassend Carbide oder Oxycarbide eines Nebengruppenelements, sind allgemein bekannt.

Bevorzugt umfasst der Katalysator Molybdäncarbid, Molybdänoxicarbid, Wolframcarbid, Wolframoxicarbid und Mischungen der vorgenannten Verbindungen.

Bevorzugt setzt man als heterogenen Katalysator einen Trägerkatalysator ein, bei dem ein Carbid oder Oxycarbid eines Nebengruppenelements die Aktivkomponente (Aktivator A) bildet, die auf einen üblichen Träger (Träger T) aufgebracht ist. Der Anteil des Aktivators (A) am Katalysator (K) beträgt üblicherweise 0,1 bis 30 Gew.-%.

Als Träger (Träger T) für die Herstellung der Katalysatoren kommen alle üblicherweise für die Herstellung von Trägerkatalysatoren verwendeten Materialien in Betracht wie Metalloxide, -nitride, -boride, -carbide, -silicate, Aktivkohle, Graphit. Bevorzugt handelt es sich um Verbindungen von Elementen der Hauptgruppen oder der VI. oder II. Nebengruppe sowie Mischungen der vorgenannten Verbindungen. Besonders bevorzugt sind Al₂O₃, Alumosilicate, Ga₂O₃, SiO₂, GeO₂, TiO₂, ZrO₂, SnO₂ und Mischungen der vorgenannten Verbindungen. Geeignete Träger weisen typischerweise ein spezifische Oberfläche von 10 - 500 m²/g, bevorzugt 100 - 400 m²/g auf. Das bevorzugte Porenvolumen (bestimmt mittels Quecksilberporsosimetrie) beträgt 0,3 bis 1,3 ml/g. Die bevorzugte Wasseraufnahme beträgt 0,5 bis 1,5 ml/g. Bei dem Träger handelt es sich üblicherweise um Formkörper wie Kugeln, Splitt, Stränge oder Tabletten. Der Träger kann gegebenenfalls zusätzlich mit Säuren, Basen oder Alkoholen vorbehandelt sein.

Die Herstellung der üblichen Trägerkatalysatoren mit Carbiden oder Oxicarbiden als Aktivator (A) nimmt man üblicherweise vor, indem man
a.1) den Träger (T) mit einer Lösung einer Verbindung eines Nebengruppenelements imprägniert (Schritt a.1)
b.1) den gemäß Schritt a. 1 imprägnierten Träger (T) anschließend trocknet und danach calciniert (Schritt b.1),
c.1) den gemäß Schritt b.1 behandelten Träger (T) bei einer Temperatur von 550 bis 1000°C in einer Atmosphäre, enthaltend eine Kohlenwasserstoffverbindung und Wasserstoff, tempert (Schritt c.1).

In Schritt (b.1) werden Katalysator-Precursoren gebildet, die als Aktivkomponenten Oxide tragen. Die Herstellung solcher Katalysator-Precursoren ist allgemein bekannt. Beispielsweise imprägniert man in Schritt (a.1) den Träger T mit einer Lösung von Verbindungen des entsprechenden Nebengruppenelements. Solche Verbindungen sind z.B. organische Komplexe, Halogenide, Säuren, Polysäuren, Heteropolysäuren und Salze der Säuren, Polysäuren und Heteropolysäuren. Solche Salze sind bevorzugt Alkali- oder Ammoniumsalze. Als organische Komplexe werden in diesem Zusammenhang beispielsweise Dialkylkomplexe, Acylverbindungen, Acetylacteonate, oder Allylkomplexe verstanden. Im Fall von Wolframoxid dient hierzu z.B. eine Lösung von Ammoniummetawolframat, Wolframsäure oder Wolframpentachlorid. Die imprägnierten Träger werden anschließend meistens mehrere Stunden bei Temperaturen von 100 bis 200°C an der Luft getrocknet. Danach folgt üblicherweise ein Calcinierungsschritt. Hierzu werden die imprägnierten und getrockneten Träger üblicherweise in einer Sauerstoff-haltigen Gasatmosphäre, z.B. an der Luft, auf Temperaturen von 400 bis 850°C über einen Zeitraum von etwa einer halben Stunde bis 5 Stunden erhitzt. Die derart hergestellten Katalysator-Precursoren können auch noch mit Temperschritten in Inertgas, beispielsweise N₂, CO₂ oder Edelgase, vorbehandelt werden oder in reduzierenden Gasmischungen, die beispielsweise Wasserstoff, CO, Ammoniak oder Hydrazin enthalten, partiell reduziert werden.

Zur Carbidisierung (Schritt c.1) werden die entsprechenden metalloxidtragenden Katalysator-Precursoren in einem kohlenwasserstoffhaltigen Strom, z.B. einem Methanstrom, in Gegenwart von Wasserstoff im Allgemeinen mehrere Stunden bei Temperaturen von 550 bis 800°C erhitzt. Zur Herstellung der Wolframcarbide werden typischerweise um etwa 50 - 200 °C höhere Temperaturen als zur Herstellung der Molybdäncarbide benötigt. Die Eigenschaften der Carbide werden auch durch das H₂/CH₄-Verhältnis beeinflusst, ein typischer Wert liegt hier bei 80/20. Die entsprechenden Carbidisierungsmethoden sind bekannt und z.B. in Oyama, S. T., Catal. Today, 15 (1992), 179 beschrieben.

Nach der Carbidisierung müssen diese Katalysator-Precursoren wegen ihrer Luftempfindlichkeit unter Inertgasatmosphäre gelagert werden, oder mit verdünntem Sauerstoff passiviert und dann im Synthesereaktor reaktiviert werden. Eine weitere Möglichkeit besteht in dem Ausbau der frisch hergestellten Carbide unter einer Flüssigkeit, die die Carbidoberfläche vor Luftsauerstoff weitgehend schützt.

Weiterhin eignen sich folgende Verfahren zur Herstellung von Katalysator-Precursoren, die als Aktivator-Precursoren Carbide tragen:

Lee et al. beschreiben in J. Catal. 128, 126 (1991) die Herstellung von Al₂O₃geträgerten Molybdäncarbiden durch (i) Reduktion gefolgt von Carbidisierung, (ii) direkte Carbidisierung in CH₄/H₂ oder (iii) Nitridierung mit NH₃ gefolgt von der Carbidisierung.

In Volpe, Boudart, J. Solid State Chem. 59, 332 (1985) und Volpe, Boudart, J. Solid State Chem. 59, 348 (1985) wird die Nitridierung/Carbidisierung von MoO₃ und WO₃ näher beschrieben.

Die Reduktion von MoO₃ auf Kohle-Trägern durch Wasserstoff, die mit einer Carbidisierung durch den Kohleträger ab Temperaturen von 530°C gekoppelt ist, wird etwa in Liang et al., Chem. Mater. 2002,14, 3148 beschrieben.

Was die Oxycarbide betrifft, die als Aktivator-Precursoren in Betracht kommen, so sind diese z.B.: in Pham-Huu et al., Appl. Catal. A 132 (1995), 77 beschrieben. Die Herstellung kann entweder aus den Oxiden durch nur teilweise Carbidisierung erfolgen. Die Oxicarbide bilden sich unter geeigneten Bedingungen auch während der Reaktion, wenn vom Oxid ausgegangen wird und dann ein Kohlenwasserstoff/H₂-Gemisch über den Katalysator bei erhöhten Temperaturen geleitet wird (etwa: H₂/n-Hexan = 150, T = 350°C).

Die Oxicarbide können auch hergestellt werden durch Sauerstoffbehandlung von Carbiden. In Ledoux et al., New Frontiers in Catalysis, 1993, S. 955, Guczi, L. et al (Editors), Elsevier Science Publishers B.V., wird das Carbid bei 350°C erst mit Luft und dann bei derselben Temperatur mit Wasserstoff behandelt.

Besonders gute Ergebnisse erzielt man mit aktivierten Katalysatoren, die erhältlich sind, indem man
a.2) auf einen üblichen Träger ein Carbid oder Oxycarbid eines Nebengruppenelements aufbringt und so einen Katalysator-Precursor (a2) herstellt (Schritt a.2)
b.2) den Katalysator-Precursor (a.2) mit einer Kohlenwasserstoffverbindung bei einer Temperatur zwischen -20 und 550°C in Kontakt bringt (Schritt b.2) und
c.2) den gemäß Schritt (b.2) hergestellten Katalysator-Precursor in einer Inertgasatmosphäre bei einer Temperatur von 410 bis 850 C°tempert (Schritt c.2).

Schritt (a.2) wird dabei im Allgemeinen so vollzogen wie vorstehend für die üblichen heterogene Katalysatoren, umfassend Carbide oder Oxycarbide eines Nebengruppenelements, beschrieben, bevorzugt nach den Schritten (a.1) bis (c.1). Die erfindungsgemäß einzusetzenden üblichen heterogenen Katalysatoren, umfassend Carbide oder Oxycarbide eines Nebengruppenelements, dienen also in Schritt (a.2) als Katalysator-Precursoren (a.2) zur Herstellung der aktivierten Katalysatoren, die im erfindungsgemäßen Verfahren besonders bevorzugt eingesetzt werden.

Gemäß Schritt (b.2) werden die Katalysator-Precursoren (a.2) mit einer Kohlenwasserstoffverbindung in Kontakt gebracht. Geeignete Kohlenwasserstoffverbindungen sind vor allem Aromaten, Alkane, Cycloalkane, Alkine, Cycloalkine, Olefine oder Cycloolefine mit 1 bis 20 Kohlenstoffatomen. Besonders bevorzugt sind C₃- bis C₁₂-Olefine, ganz besonders Butene und Oktene, wie z.B. 1-Buten und n-Okten-1.

Bei der Behandlung des Katalysator-Precursors mit der Kohlenwasserstoffverbindung kann diese sowohl flüssig als auch gasförmig vorliegen. Die Behandlungsdauer ist unkritisch und beträgt üblicherweise 1 min - 24 h, bevorzugt 5 min - 4 h. Die Temperatur während der Behandlung beträgt im Allgemeinen -20 bis 550°C, sie ist jedoch unkritisch. Letzteres gilt auch für den Druck, er beträgt im Allgemeinen 0,5 bis 40 bar.

Der mit der Kohlenwasserstoff vorbehandelte Katalysator-Precursor wird anschließend in Schritt (c.2) in einer Inertgasatmosphäre auf eine Temperatur von 410 bis 850, bevorzugt 500 bis 850°C erhitzt. Geeignete Inertgase sind vor allem Stickstoff, CO₂ sowie die Edelgase. Die Behandlung in Schritt c) erfolgt üblicherweise über einen Zeitraum von 5 min bis 100 h, bevorzugt 30 min bis 24 h, wobei der Druck hier wiederum ebenfalls unkritisch ist und üblicherweise 0,5 bis 40 bar beträgt.

Die eingesetzten Katalysatoren können zusätzlich auch Promotoren enthalten. Hierbei handelt es im Allgemeinen um Cobalt-, Alkali-oder Eralkalimetallverbindungen. Sie werden im Allgemeinen auf den Katalysator aufgebracht, indem man den Tränklösungen in Schritt (a.1) zur Herstellung der Katalysator-Precursoren entsprechende Salze, z.B. Nitrate oder Hydroxide beifügt oder die Katalysatoren mit einer entsprechenden Tränklösung nachträglich dotiert und zur Fixierung nochmals calciniert.

Das erfindungsgemäße Verfahren zur Herstellung von einer Verbindung mit einer nicht-aromatischen C-C-Doppel oder Dreifachbindung (Verbindung A) aus einer anderen Verbindung oder einer Mischung anderer Verbindungen mit einer nicht-aromatischen C-C-Doppel- oder Dreifachfindung (Verbindung B). Der Begriff Verbindung (B) steht in diesem Text also nicht nur für eine einzelne Verbindung sondern auch für eine Mischung verschiedener Verbindungen, was zu einer Kreuzmetathese führt.

Als Verbindungen (B) werden günstigerweise entsprechende Kohlenwasserstoffe mit 2 bis 12 Kohlenstoffatomen eingesetzt. Bevorzugt ist die Verbindung (B) ausgewählt aus der Gruppe C₂- bis C₁₂-Olefine, substituierte C₂- bis C₁₂-Olefine und Mischungen der vorgenannten Verbindungen. Unter substituierten Olefinen werden in diesem Zusammenhang beispielsweise ungesättigte Säuren, Ester, Nitrile, Halogenide, Ketone, Aldehyde oder Amine verstanden.

Besonders günstig können die erfindungsgemäßen Katalysatoren in Metatheseverfahren zur Herstellung von Propen durch Metathese einer Mischung, die 2-Buten und Ethylen oder 1-Buten und 2-Butene enthält, sowie von 3-Hexen und Ethylen durch Metathese von 1-Buten eingesetzt werden. Entsprechende Verfahren sind im Detail in DE-A-19813720, EP-A-1134271, WO 02/083609, DE-A-10143160 beschrieben.

Die vorgenannten C₄-Ausgangsverbindungen werden üblicherweise in Form eines sogenannten Raffinat II bereitgestellt. Bei dem Raffinat II handelt es sich um C₄-Schnitte, die im Allgemeinen einen Gehalt an Butenen von 30 bis 100, bevorzugt 40 bis 98 Gew.-% aufweisen. Neben den Butenen können vor allem noch gesättigte C₄-Alkane vorhanden sein. Die Gewinnung solcher Raffinate II ist allgemein bekannt und z.B. in der EP-A-1134271 beschrieben.

Insbesondere kann 1-Buten eingesetzt werden, das durch Abdestillieren einer 1-Buten reichen Fraktion aus Raffinat II, gewonnen wird. Aus der verbleibenden, an 2-Butenen reichen Fraktion kann ebenfalls 1-Buten gewonnen werden, indem man die 2-Buten reiche Fraktion einer Isomerisierungsreaktion unterwirft und anschließend destillativ in eine 1-Buten und eine 2-Buten reiche Fraktion auftrennt. Dieses Verfahren ist in der DE-A-10311139 beschrieben.

Das erfindungsgemäße Verfahren wird im Allgemeinen kontinuierlich in der Gasphase durchgeführt. Die Temperatur liegt hier im Allgemeinen bei 100 bis 500°C. Der Druck beträgt im Allgemeinen 5 - 50 bar.

Die Belastung des Katalysators beträgt im Allgemeinen 1 bis 30 g, bevorzugt 5 bis 20 g Verbindung (B) pro g Katalysator pro h.

### Experimenteller Teil

### A. Herstellung der eingesetzten Katalysatoren

### Herstellung eines WO₃/SiO₂-Katalysators - Kat A

30 g Ammoniummetawolframat (W-Gehalt 72,2%) wurden mit H₂O auf 190 ml verdünnt. Diese Lösung wurde auf 200 g eines SiO₂-Trägers (BASF D11-10, 1.5 mm Stränge, 171 m²/g) aufgetränkt. Danach wurden die Stränge 16 h im Trockenschrank bei 120°C getrocknet. Abschließend wurde der Katalysator am Drehrohrofen bei 593°C in Luft (20 I/h) 1 h lang calciniert und unter trockenem Stickstoff abgekühlt. Der WO₃-Gehalt betrug 12,1 wt%.

XRDs von Ausbaukatalysatoren dieses Typs lassen nach der beschriebenen Metathesereaktion leicht unterstöchimetrische WOₓ-Phasen erkennen, vorwiegend WO_{2.92}. Es konnten in keinem Fall carbidische Spezies nachgewiesen werden.

### Herstellung eines Wolframcarbid-Katalysators - Kat B

Zur Herstellung von Katalysator B wurde in einen von oben nach unten durchströmten Glasreaktor 70 ml des WO₃/SiO₂-Katalysators A eingefüllt. Der Glasreaktor wurde von außen durch einen elektrischen Ofen beheizt, die Katalysatorschüttung befand sich etwa in der Mitte der Heizzone auf einer Glasfritte. Nach Einbau des Katalysators und Verschließen des Reaktors wurde die Anlage zunächst mit Stickstoff gespült (30 min, 20 Uh). Anschließend wurde ein Gasstrom bestehend aus 3,9 L/h Methan und 15 L/h Wasserstoff über den Katalysator geleitet. Der Reaktor wurde dann innerhalb 180 min auf 750°C erhitzt und 6 h bei 750°C gehalten. Danach wurde innerhalb 1 h auf 500°C abgekühlt und diese Temperatur 2 h gehalten. Dann wurde abgekühlt und der Methan/Wasserstoffstrom durch einen Stickstoffstrom ersetzt. Nach erfolgter Spülung des Reaktors wurde der Reaktorein- und Ausgang verschlossen und der Reaktor aus der Anlage ausgebaut, so daß der Katalysator ohne Kontakt mit Luft in eine Glove-Box transferiert werden konnte. Bei dem weiteren Katalysatorhandling wie etwa dem Einbau des Katalysators in den Reaktor bzw. in die Analysengeräte wurde ebenfalls ein Kontakt mit Luft vermieden.

Ein XRD (X-Ray diffraction) des Ausbaukatalysators nach der Metathese-Reaktion zeigt die Verbindungen WC und W₂C, daneben Spuren von metallischem Wolfram. WOₓ-Verbindungen sind nicht zu erkennen.

### Herstellung eines Wolframcarbid-Katalysators - Kat C

Zur Herstellung von Katalysator C wurde in einen von oben nach unten durchströmten Glasreaktor 70 ml des WO₃/SiO₂-Katalysators A eingefüllt. Der Glasreaktor wurde von außen durch einen elektrischen Ofen beheizt, die Katalysatorschüttung befand sich etwa in der Mitte der Heizzone auf einer Glasfritte. Nach Einbau des Katalysators und Verschließen des Reaktors wurde die Anlage zunächst mit Stickstoff gespült (30 min, 20 L/h). Anschließend wurde ein Gasstrom bestehend aus 3,9 L/h Propylen und 15 L/h Wasserstoff über den Katalysator geleitet. Der Reaktor wurde dann innerhalb 60 min auf 400°C erhitzt, dann innerhalb von 120 min auf 650°C erhitzt und 5,5 h bei 650°C gehalten. Dann wurde abgekühlt und der Propylen/Wasserstoffstrom durch einen Stickstoffstrom ersetzt. Nach erfolgter Spülung des Reaktors wurde der Reaktoreinund Ausgang verschlossen und der Reaktor aus der Anlage ausgebaut, so daß der Katalysator ohne Kontakt mit Luft in eine Glove-Box transferiert werden konnte. Bei dem weiteren Katalysatorhandling wie etwa dem Einbau des Katalysators in den Reaktor bzw. in die Analysengeräte wurde ebenfalls ein Kontakt mit Luft vermieden.

Das XRD (X-Ray diffraction) des Ausbaukatalysators nach der Metathesereaktion zeigt eine eher amorphe Struktur. Es kann W₂C identifiziert werden, daneben ist eine Spezies vorhanden, die möglicherweise W₂N zuzuordnen ist. WOₓ-Verbindungen sind nicht zu erkennen.

### B. Metatheseverfahren

### Metathese von 1-Buten mit aktivierten Katalysatoren A, B und C

Ca. 35 g Katalysator A, B und C wurden jeweils in einen elektrisch beheizten Rohrreaktor gefüllt. Am Eingang des Katalysatorbetts wurde eine Temperatur von 190°C eingestellt. Eine ungleichmäßige Temperaturverteilung der Heizung führte zu einem Anstieg der Temperatur hin zum Ende des Katalysatorbetts (Maximale Temperatur ist jeweils in Klammern angegeben). Als Feed wurde reines 1-Buten zugefahren. Der Reaktionsdruck war 9,7 bar. Die Analyse des Reaktoraustrags erfolgte on-line mit einem GC. Vor der eigentlichen Messung waren alle Katalysatoren nach folgender Prozedur aktiviert worden: Unter Reaktionsbedingungen für 3-4 h mit 1-Buten anfahren und anschließend unter fließendem Stickstoff (30 I/h) bei ca. 500°C Innentemperatur 17 h lang tempern. Die Versuchsergebnisse können Tabelle 1 entnommen werden.

**Tabelle 1:**

| Kat | T [°C] | WHSV h⁻¹ | 1-Buten-Umsatz [%] | Isomerisierung ¹⁾ [%] | Hexene [mol%] | Propen [mol%] | C₆-Selektivität [mol%] |
|---|---|---|---|---|---|---|---|
| A (Vgl.) | 189 (274) | 8,0 | 63,0 | 32,7 | 11,5 | 16,1 | 36,4 |
| B | 155 (238) | 4,3 | 44,1 | 13,9 | 13,5 | 6,9 | 61,1 |
| B | 190 (293) | 8,8 | 52,8 | 22,5 | 13,4 | 10,2 | 50,8 |
| C | 190 (240) | 8,8 | 48,9 | 22,2 | 11,4 | 9,1 | 46,7 |

| | | | | | | | |
|---|---|---|---|---|---|---|---|
| ¹⁾ Summe der gebildeten Allylfragmente = [Propen]/2 + 2-Butene + Pentene | | | | | | | |

### Metathese von 1-Buten mit nicht-aktivierten Katalysatoren A, B und C

Die Reaktion wurde wie vorstehend beschrieben durchgeführt, allerdings wurde auf die dort beschriebene Aktivierungsprozedur verzichtet und die Katalysatoren nach Einbau direkt mit 1-Buten bei Reaktionstemperatur angefahren. Die Versuchsergebnisse können Tabelle 2 entnommen werden.

**Tabelle 2:**

| Kat | T [°C] | WHSV [h⁻¹] | Rate (Hexen) [mol/l_{Kat}*h] | Rate (Propen) [mol/l_{Kat}*h] |
|---|---|---|---|---|
| B | 190 (303) | 8,8 | 0,16 | 0,70 |
| C | 190 (250) | 8,8 | 0,56 | 0,37 |
| C | 190 (253) | 13,2 | 0,30 | 0,75 |

Bei der Nachstellung des Versuchs mit einem Vergleichskatalysator, der gemäß Beispiel 1 hergestellt wurde und einen WO₃-Gehalt von 13,9 wt% aufwies (WHSV = 7,9; T = 190 (256°C)), war keinerlei Hexen oder Propen nachzuweisen.

## Patentansprüche

1. Verfahren zur Herstellung von einer Verbindung mit einer nicht-aromatischen C-C-Doppel oder Dreifachbindung (Verbindung A) aus einer anderen Verbindung oder einer Mischung anderer Verbindungen mit einer nicht-aromatischen C-C-Doppel oder Dreifachfindung (Verbindung B), wobei man die Verbindung (B) bei einer Temperatur von 50 bis 500°C mit einem heterogenen Katalysator, umfassend Carbide oder Oxycarbide eines Nebengruppenelements, in Kontakt bringt.

2. Verfahren nach Anspruch 1, wobei die Verbindung (B) ausgewählt ist aus der Gruppe C₂- bis C₁₂-Olefine, substituierte C₂- bis C₁₂-Olefine und Mischungen der vorgenannten Verbindungen.

3. Verfahren nach einem der vorstehenden Ansprüche, wobei der heterogene Katalysator ausgewählt ist aus der Gruppe bestehend aus Molybdäncarbid, Molybdänoxicarbid, Wolframcarbid, Wolframoxicarbid und Mischungen der vorgenannten Verbindungen.

4. Verfahren nach einem der vorstehenden Ansprüche, wobei man als heterogenen Katalysator einen Trägerkatalysator einsetzt, bei dem ein Carbid oder Oxycarbid eines Nebengruppenelements die Aktivkomponente (Aktivator A) bildet, die auf einen üblichen Träger (Träger T) aufgebracht ist.

5. Verfahren nach Anspruch 4, wobei der Anteil des Aktivators (A) an dem Trägerkatalysator 0,1 bis 30 Gew.-% beträgt.

6. Verfahren nach Anspruch 4 oder 5, wobei man als heterogenen Katalysator einen Trägerkatalysator einsetzt, dessen Träger (T) ausgewählt ist aus der Gruppe bestehend aus Al₂O₃, Alumosilicate, Ga₂O₃, SiO₂, GeO₂, TiO₂, ZrO₂, SnO₂ und Mischungen der vorgenannten Verbindungen.

7. Verfahren nach einem der Ansprüche 4 bis 6, wobei man den Trägerkatalysator herstellt indem man
a.1) den Träger (T) mit einer Lösung einer Verbindung eines Nebengruppenelements imprägniert (Schritt a.1)
b.1) den gemäß Schritt a.1 imprägnierten Träger (T) anschließend trocknet und danach calciniert (Schritt b.1),
c.1) den gemäß Schritt b.1 behandelten Träger (T) bei einer Temperatur von 550 bis 1000°C in einer Atmosphäre, enthaltend eine Kohlenwasserstoffverbindung und Wasserstoff, tempert (Schritt c.1).

8. Verfahren nach einem der Ansprüche 4 bis 7, wobei man als heterogenen Katalysator einen Trägerkatalysator einsetzt, der erhältlich ist, indem man
a.2) auf einen üblichen Träger ein Carbid oder Oxycarbid eines Nebengruppenelements aufbringt und so einen Katalysator-Precursor (a2) herstellt (Schritt a.2)
b.2) den Katalysator-Precursor (a.2) mit einer Kohlenwasserstoffverbindung bei einer Temperatur zwischen -20 und 550°C in Kontakt bringt (Schritt b.2) und
c.2) den gemäß Schritt (b.2) hergestellten Katalysator-Precursor in einer Inertgasatmosphäre bei einer Temperatur von 410 bis 850 C°tempert (Schritt c.2).

9. Verfahren nach einem der Ansprüche 4 bis 8, wobei man in Schritt (b.2) eine Kohlenwasserstoffverbindung, ausgewählt aus der Gruppe bestehend aus C₁- bis C₂₀-Alkanen, -Cycloalkanen, -Olefinen, -Cycloolefinen, -Alkinen, -Cycloalkinen, Aromaten und Mischungen der vorgenannten Verbindungen, einsetzt.

10. Verfahren nach einem der Ansprüche 4 bis 9, wobei man in Schritt (c.2) ein Inertgas, ausgewählt aus der Gruppe bestehend aus Stickstoff, Kohlendioxid und Edelgas oder Mischungen hiervon einsetzt.
